# EUROPEAN PATENT SPECIFICATION

(11) **EP 0 848 991 B1**
(45) Date of publication and mention of the grant of the patent: **24.03.2004**
(21) Application number: 97310014.2
(22) Date of filing: 11.12.1997
(51) Int. Cl.: B01J 23/656, B01J 23/68, C07C 29/149, C07C 31/20, C07D 307/08, B01J 23/89

(54) **Improved catalysts for the hydrogenation of maleic acid to 1,4-butanediol**
Verbesserte Katalysatoren für die Hydrierung von Maleinsäure zu 1,4-Butandiol
Catalyseurs améliorés pour l'hydrogènation de l'acide maléique en 1,4-butanediol

(30) Priority: 20.12.1996 US 781945
(43) Date of publication of application: 24.06.1998
(73) Proprietor: The Standard Oil Company, Chicago, IL 60601 (US)
(72) Inventor: Raymond, John, Beachwood, Ohio 44122 (US); Attig, Thomas George, Aurora, Ohio 44202 (US); Dubbert, Robert Allen, Solon, Ohio 44139 (US)
(74) Representative: Hoey, Shona

(56) References cited:
- EP-A- 0 662 343
- WO-A-92/02298
- US-A- 5 473 086

## Description

This invention relates to an improved catalyst for the hydrogenation of maleic acid, maleic anhydride or other hydrogenatable precursor to 1,4-butanediol and tetrahydrofuran. The catalyst comprises palladium, silver, rhenium and iron, all on a carbon support. The use of this catalyst in processes for the hydrogenation of maleic acid, maleic anhydride or other hydrogenatable precursor to 1,4-butanediol and tetrahydrofuran is characterized by higher overall activity to reaction products and by higher yields of 1,4-butanediol with minimal formation of gamma-butyrolactone by-products.

It is well known that tetrahydrofuran, gamma-butyrolactone and 1,4-butanediol are obtained by the catalytic hydrogenation of maleic anhydride and related compounds. Tetrahydrofuran is a useful solvent for natural and synthetic resins and is a valuable intermediate in the manufacture of a number of chemicals and plastics. Gamma-butyrolactone is an intermediate for the synthesis of butyric acid compounds, polyvinylpyrrolidone and methionine. Gamma-butyrolactone is a useful solvent for acrylate and styrene polymers and also a useful ingredient of paint removers and textile assistants. 1,4-butanediol (a.k.a. 1,4-butylene glycol) is useful as a solvent, a humectant, an intermediate for plasticizers and pharmaceuticals, a cross-linking agent for polyurethane elastomers, a precursor in the manufacture of tetrahydrofuran, and is used to make terephthalate plastics.

Of particular interest in the instant invention are hydrogenation catalysts comprising palladium, silver, rhenium and iron on a carbon support, which are useful for the hydrogenation of maleic anhydride, maleic acid and related compounds to tetrahydrofuran, gamma-butyrolactone and 1,4-butanediol.

British Patent No. 1,534,232 teaches the hydrogenation of carboxylic acids, lactones or anhydrides using a hydrogenation catalyst consisting of palladium and rhenium on a carbon support. U.S. Patents 4,550,185 and 4,609,636 teach a process of making tetrahydrofuran and 1,4-butanediol by hydrogenating maleic acid, maleic anhydride or other hydrogenatable precursor in the presence of a catalyst comprising palladium and rhenium on a carbon support wherein the palladium and rhenium were present in the form of crystallites having an average palladium crystallite size of about 10 to 25 nm and an average rhenium crystallite size of less than 2.5 nm. The preparation of this catalyst is characterized by the deposition and reduction of the palladium species on the carbon support followed by the deposition and reduction of the rhenium species on the palladium impregnated carbon support.

U.S. Patent 4,985,572 teaches a process for the catalytic hydrogenation of a carboxylic acid or an anhydride thereof to the corresponding alcohol and/or carboxylic acid ester using a catalyst comprising rhenium, palladium and at least one other metal capable of alloying with the palladium, all on a carbon support. The preferred metal capable of alloying with the palladium is silver but gold, copper, nickel, rhodium, tin, cobalt, . aluminum, manganese, gallium, iron, chromium, and platinum also are taught. The preparation of this catalyst is characterized by the simultaneous deposition of palladium and silver on the carbon support followed by a high temperature (600°C) heat treatment. Rhenium is then deposited on the palladium/alloying metal impregnated carbon support. The resulting catalyst is then reduced.

WO 92/02298 discloses a hydrogenation catalyst comprising palladium and rhenium and one or more metals selected from the group consisting of rhodium, cobalt, platinum, ruthenium, iron, thulium, cerium, yttrium, neodymium, aluminum, praseodymium, holmium, hafnium, manganese, vanadium, chromium, gold, terbium, lutetium, nickel, scandium and niobium, on a support. US 5,473,086 discloses a process for the catalytic hydrogenation of maleic acid anhydride or other hydrogenatable precursors to 1,4, butanediol and tetrahydroform. The catalyst employed in the process comprises palladium, silver and rhenium on a carbon support.

Generally, in the hydrogenation of maleic acid, maleic anhydride or other hydrogenatable precursor the above discussed catalysts have the propensity to produce more tetrahydrofuran and gamma-butyrolactone than 1,4-butanediol. An object of this invention is a process and a catalyst which will maximize 1,4-butanediol production and minimize gamma-butyrolactone production.

### SUMMARY OF THE INVENTION

The instant invention is a catalyst comprising palladium, silver, rhenium and iron on a carbon support and the use of this catalyst in a process for the production of 1,4-butanediol comprising catalytically hydrogenating a hydrogenatable precursor in contact with a hydrogen-containing gas.

Another embodiment of the instant invention is a method for making such catalyst for the production of 1,4-butanediol comprising:
(i) oxidizing a carbon support by contacting the carbon support with an oxidizing agent;
(ii) impregnating in one or more impregnation steps comprising contacting a carbon support with a source of palladium, silver, rhenium and iron;
(iii) drying the impregnated carbon support to remove solvent after each impregnation step; and
(iv) heating the impregnated carbon support from ambient temperature to a temperature of between about 100° C and about 350°C under reducing conditions.

### DETAILED DESCRIPTION OF THE INVENTION

A catalyst comprising palladium, rhenium, silver and iron on a carbon support and wherein the catalyst comprises between about 0.1 to about 20 wt% palladium between about 0.1 to about 20 wt% silver, between about 0.1 to about 20wt% rhenium and between about 0.1 to about 5 wt% iron,is employed in the hydrogenation of a hydrogenatable precursor to provide high yields of 1,4-butanediol and smaller yields of tetrahydrofuran with minimal gamma-butyrolactone formation.

### The Reactants

In the process of the instant invention, at least one hydrogenatable precursor is reacted with a hydrogen containing gas in the presence of the catalyst. As used herein a "hydrogenatable precursor" is selected from the group consisting of maleic acid, maleic anhydride, fumaric acid, succinic anhydride, succinic acid, succinate esters such as dimethyl succinate, maleate esters such as dimethyl maleate, gamma-butyrolactone or mixtures thereof The preferred hydrogenatable precursors are maleic acid, maleic anhydride, succinic acid, succinic anhydride or mixtures thereof.

The most preferred hydrogenatable precursor is maleic acid which is typically obtained by reacting n-butane or benzene in an oxygen-containing gas in the presence of a catalyst to oxidize in the vapor phase the n-butane or benzene to maleic anhydride, and then collecting the maleic anhydride by a water quench to produce maleic acid in an aqueous solution. The oxidation of n-butane or benzene is typically operated at a temperature of about 300°C to 600°C and a pressure of about 0.5 to 20 atmospheres (51 to 2027 kPa).

Typically, the hydrogen (H₂) containing gas is commercially pure hydrogen with no diluent gases. However, the hydrogen containing gas in addition to hydrogen (H₂) may also contain nitrogen (N₂), any gaseous hydrocarbon (e.g. methane), as well as gaseous oxides of carbon, (e.g. carbon monoxide, carbon dioxide).

### The Catalyst

The catalyst employed in the instant invention comprises palladium, silver, rhenium and iron supported on carbon. The carbons for use in this invention have a BET surface area of at least 200 m²/g, and preferably be in the range of 500-1500 m²/g.

The catalyst composition comprises about 0.1 to about 20 weight percent palladium, preferably about 2 to about 8 weight percent palladium, more preferably about 2 to about 4 weight percent palladium; about 0.1 to about 20 weight percent silver, preferably about 1 to about 8 weight percent silver, more preferably about 2 to about 4 weight percent silver; about 0.1 to about 20 weight percent rhenium, preferably about 1 to about 10 weight percent rhenium, more preferably about 5 to about 9 weight percent rhenium; and about 0.01 to about 10 weight percent iron, preferably about 0.1 to about 5 weight percent iron, more preferably about 0.2 to about 0.6 weight percent iron. The ratio of palladium to silver is between 10 to 1 and 1 to 10. The catalyst composition may also be further modified through the incorporation of a metal or metals selected from Groups IA, IIA or VIII.

The catalysts of this invention may be conveniently prepared by oxidizing the carbon support (however this treatment step is optional) followed by impregnation of the carbon support, either in single or multiple impregnation steps, with a solution or solutions containing at least one palladium, silver, rhenium or iron compound.

Preferably, the carbon support is first oxidized by contacting the carbon support, prior to deposition of the metals, with a oxidizing agent. Catalysts prepared in this manner show a dramatic improvement in activity and selectivity over catalysts prepared with non-oxidized carbon support. A number of oxidizing agents such as nitric acid, hydrogen peroxide, sodium hypochlorite, ammonium persulfate, perchloric acid, and oxygen may be effective in this process. Liquid phase oxidizing agents are preferred. Nitric acid at elevated temperatures has been found to be especially effective for this procedure. Gaseous phase oxidizing agents include any oxygen-containing gas, e.g. air. Gaseous oxidizing agents are contacted with the carbon support at temperatures of about 200°C or greater and at pressures of about atmospheric or greater. Optionally one or more metals, such as iron, nickel, palladium, rhenium, silver, gold copper, rhodium, tin, cobalt, manganese, gallium, and platinum, may be admixed with the oxidizing agent and subsequently deposited on the carbon support during the oxidizing agent pretreatment of the carbon support.

As stated earlier, the catalysts of this invention are prepared by impregnation of the carbon support, either in single or multiple impregnation steps, with a solution or solutions containing at least one palladium, silver, rhenium or iron compound. As used herein, impregnation of the carbon support means to cause the carbon support to be filled, imbued, permeated, saturated or coated. The impregnating solution may optionally contain complexing agents to help solubilize one or more of the metal compounds. The impregnating solution may also optionally be combined with the oxidizing agent prior to or in situ with contacting the carbon support. The catalyst is dried after each impregnation step to remove any carrier solvent. Drying temperatures are between about 80°C and about 150°C.

The solutions of palladium compound, silver compound, rhenium compound and iron compound can be applied to the carbon by immersing or suspending the support material in the solution or by spraying the solution onto the carbon. The solution containing the palladium compound is typically an aqueous solution containing an amount of palladium compound to yield a catalyst product with the requisite amount of palladium. The palladium compound may be palladium nitrate or a palladium compound such as a chloride, carbonate, carboxylate, acetate, acetyl acetonate, or amine. The solution containing the silver compound is typically an aqueous one containing an amount of silver compound to yield a catalyst product with the requisite amount of silver. The palladium and silver compounds should be thermally decomposable and reducible to the metals. The solution containing the rhenium compound is typically an aqueous one containing an amount of rhenium compound to yield a catalyst product with the requisite amount of rhenium. The rhenium compound is typically perrhenic acid, ammonium perrhenate or an alkali metal perrhenate. The solution containing the iron compound is typically an aqueous one containing an amount of iron compound to yield a catalyst product with the requisite amount of iron. The iron compound is typically ferric nitrate, but other suitable iron containing compounds include, but are not limited to, ferrous acetate, ferric acetate, ferrous chloride, ferrous fumarate, and ferric fumarate.

The impregnating solution(s) may optionally contain metal complexing agents to help solubilize one or more of the metal compounds. The addition of acetonitrile to the impregnating solution allows the Pd, Ag, and Re compounds be added in a single step. Nitric acid or other oxidizing agent may also be added to the impregnating solution.

After impregnation with palladium, silver, rhenium and iron and drying, the catalyst is activated by heating the impregnated carbon support under reducing conditions from ambient temperature (i.e. typically room temperature) to a temperature of between about 120°C and 350°C, preferably between about 150°C and about 300°C. Hydrogen, or a mixture of hydrogen and nitrogen, in contact with the catalyst may be conveniently used for the catalyst reduction. Reduction of the impregnated carbon support is only after the carbon support has been impregnated with palladium, silver, rhenium and iron. In the case of multiple impregnation steps and multiple dryings, the reduction of the catalyst is done after the final drying.

The palladium in catalysts of the present invention is present in the form of crystallites having an average crystallite size of less than 100 angstroms (10 nm) More specifically, when freshly reduced samples of the palladium/silver/rhenium on a carbon support as used herein are analyzed by X-ray diffraction (XRD) and Scanning Transmission Electron Microscopy (STEM), the palladium containing particles (i.e. particles of palladium, particles of palladium and silver, or particles of palladium and rhenium) in the catalyst are finely dispersed and have a mean crystallite size of less than about 50 angstroms (5 nm). As used herein the "particle size distribution" and "mean particle size" are as defined in "Structure of Metal Catalysts" by J. R. Anderson, pages 358-359, Academic Press (1975), which is incorporated herein by reference.

Lastly the preparation of the catalysts described herein does not employ large amounts of excess water which must be removed during the drying step nor does it employ a high temperature (i.e. about 600°C) treatment step as taught in U.S. Patent 4,985,572.

Upon completion of the catalyst preparation described herein, iron is present in the catalyst. However, when the catalyst is exposed to reducing conditions, e.g. during the hydrogenation of maleic acid, and then reexamined, no iron or iron compounds can be detected. However the catalyst still contains palladium, silver and rhenium as described herein but provides superior results in the hydrogenation of maleic acid to 1,4-butanediol compared to Pd/Ag/Re on carbon catalysts prepared without the iron component.

### The Process

The method for carrying out the process comprises reacting a hydrogenatable precursor with a hydrogen-containing gas in the presence of the hydrogenation catalyst, and recovering and purifying the reaction products by distillation.

The liquid phase hydrogenation of this invention can be run using conventional apparatus and techniques in a stirred-tank reactor or in a fixed-bed reactor. Single or multiple-stage reactors may be employed. The amount of catalyst required will vary widely and is dependent upon a number of factors such as reactor size and design, contact time and the like.

The hydrogen-containing gas is fed continuously, generally with the hydrogen in considerable stoichiometric excess to the other reactants. Unreacted hydrogen can be returned to the reactor as a recycle stream. The precursor solution, e.g., maleic acid solution, is fed continuously at concentrations ranging from dilute solutions to near the maximum solubility level, typically about 30 to about 50 weight percent.

The hydrogenation step is run at a temperature of about 50°C to 350°C, and a hydrogen pressure of about 20-400 atmospheres with hydrogen to hydrogenatable precursor ratios (H₂/P) of between 5 to 1 and 1000 to 1 and contact times of 0.1 minute to 20 hours. For maximum 1,4-butanediol production the reaction temperature is between about 50°C and 250°C and more preferably between about 80°C and 200°C.

The reaction products, 1,4-butanediol, tetrahydrofuran, gamma-butyrolactone or mixtures thereof, are advantageously separated by fractional distillation. By-products which are formed in small amounts or unreacted feed, such as for example, succinic anhydride or succinic acid, are optionally returned to the hydrogenation stage. The gamma-butyrolactone may also be recycled to the hydrogenation reactor.

Using the process of this invention, more specifically using the hydrogenation catalyst described herein, maleic acid is converted virtually quantitatively in a simple reaction. The yields of 1,4-butanediol and tetrahydrofuran achieved are about 80 mole percent or greater, typically about 90 mole percent or greater, with a majority portion of the yield being 1,4-butanediol. Reaction by-products may include n-butanol, n-butyric acid, n-propanol, propionic acid, methane, propane, n-butane, carbon monoxide, and carbon dioxide. However, the formation of non-utilizable by-products is slight.

### SPECIFIC EMBODIMENTS

In order to illustrate the instant invention the following examples are provided.

### Comparative Example A: Preparation of PdAgRe on carbon

45g of concentrated nitric acid (70 wt%) was diluted to 50 cc with deionized water. This solution was used to impregnate 74.6g of CECA 1.5 mm ACL40 carbon extrudate. During the impregnation the flask was occasionally cooled. The mixture was allowed to stand for 80 minutes, and then dried at 130°C for 3h. This procedure was repeafed with a 35 minute standing time and a 16h drying time.

35.1g of palladium nitrate solution (8.5 wt% Pd), 11.95g of perrhenic acid (53.3 wt% Re) solution and 7.9g of concentrated (70 wt% nitric acid), were diluted to 50 cc with deionized water. The ACL40 was then gradually impregnated with the Pd+Re solution. The flask was occasionally cooled during the impregnation. The mixture was allowed to stand for 2h, and then dried at 130°C for 2h.

4.7g of silver nitrate and 7.9g of concentrated nitric acid were diluted to 50 cc with deionized water. The PdRe/ACL40 was then gradually impregnated with the silver nitrate solution with occasional cooling of the flask. The mixture was allowed to stand for 3.5h, and then dried at 130C for 64h. The resulting catalyst was 3.3 wt% Pd/ 3.3 wt% Ag/ 7.1 wt% Re.

### Example 1: Preparation of PdAgReFe on carbon

45g of concentrated nitric acid (70 wt%) and 1g of ferric nitrate (Fe(NO₃)₃.9H₂O) were diluted to 50 cc with deionized water. This solution was used to impregnate 74.6g of CECA 1.5 mm ACL40 carbon extrudate. During the impregnation the flask was occasionally cooled. The mixture was allowed to stand for 65 minutes, and then dried at 130°C for 2h. This procedure was repeated with a 65 minute standing time and a 2.4h drying time.

35.1 of palladium nitrate solution (8.5 wt% Pd), 11.95g of perrhenic acid (53.3 wt% Re) solution, 7.9g of concentrated nitric acid (70 wt%), were diluted to 50 cc with deionized water. The ACL40 was then gradually impregnated with the Pd/Re solution. The flask was occasionally cooled during the impregnation. The mixture was allowed to stand for 2.5h, and then dried at 130°C for 2.25h.

4.7g of silver nitrate and 7.9g of concentrated nitric acid were diluted to 50 cc with deionized water. The PdRe/ACL40 was then gradually impregnated with the silver nitrate solution with occasional cooling of the flask. The mixture was allowed to stand for 80 minutes, and then dried at 130°C for 69h. The resulting catalyst was 3.3 wt% Pd/ 3.3 wt% Ag/ 7. 1 wt% Re/ 0.3 wt% Fe.

### Example 2: Hydrogenation of Aqueous Maleic Acid and Catalyst Testing

The catalyst of Comparative Example A and Example 1 were each tested in two Hastelloy C276 reactors connected in series using heated Hastelloy C276 tubing. The reactors had an internal diameter of 0.013m (0.516"), and each was fitted with a 0.0032m (1/8") axial Hastelloy C276 thermowell.

Each catalyst was mixed with 50/70 mesh quartz chips (0.625g quartz per g of catalyst) before charging to the reactor. 20 cc (12.15g) of catalyst was placed in the first reactor, and 40 cc (24.3g) in the second reactor. Prior to testing the catalyst was reduced at atmospheric pressure in flowing hydrogen (400 sccm) with the following temperature ramp:
Room Temperature to 30°C over 5 hours
30°C to 100°C over 2 hours
100°C to 230°C over 11 hours
maintain 230°C for 5 hours
The reactors were operated with hydrogen recycle. A small portion of the hydrogen was vented to prevent the accumulation of non-condensable gases. The maleic acid concentration in the liquid feed was 35.5 wt%. The process conditions for the catalyst testing were as following process conditions:
- Pressure: 17.2×10⁶ Pa (2500 psig)
- H₂/Maleic Acid Feed Ratio: 88
- H₂ Make-up to Recycle Ratio: 0.083
- First Reactor:
   Average Set Temperature: 100°C
   LHSV; 1.6h⁻¹
- Second Reactor:
   Average Set Temperature: 153-162°C
   LHSV: 0.8h⁻¹
Table 1 summarizes the results of the testing on the PdAgRe/C and PdAgReFe/C catalysts Table 1. Product selectivity's were calculated on a molar C₄ basis.

**Table 1 -**

| Catalyst Performance Data | | | | | | | |
|---|---|---|---|---|---|---|---|
| Catalyst | Hrs. on Stream | Temp. (C) | % BDO | %THF | %GBL | %BuOH | %SAC |
| PdAgReFe/C (Ex. 1) | 185 | 153 | 89.5 | 5.6 | 0.6 | 4.0 | 0.05 |
| PdAgRe/C (Comp. Ex) | 185 | 162 | 86.3 | 8.8 | 0.9 | 3.8 | 0.08 |

Table 1 illustrates that the BDO yield is significantly higher for PdAgReFe/C. Table 1 also illustrates that the iron containing catalyst (Example 1) is more active than the non iron containing species (Comparative Example). This is evidenced by better overall conversions at the lower reaction temperature.

It is to be understood that the subject invention is not to be limited by the examples set forth herein. These have been provided merely to demonstrate operability, and the selection of catalysts, metal sources, carbon supports, concentrations, contact times, solids loadings, feedstocks, reaction conditions, and products, if any, can be determined from the total specification disclosure provided, without departing from the spirit of the invention herein disclosed and described, the scope of the invention including modifications and variations that fall within the scope of the attached claims.

## Claims

1. A catalyst consisting essentially of palladium, silver, rhenium and iron on a carbon support wherein the catalyst comprises between 0.1 to 20 wt % palladium, between 0.1 to 20 wt % silver, between 0.1 to 20 wt % rhenium, and between 0.1 to 5 wt % iron.

2. The catalyst as claimed in claim 1 wherein the catalyst comprises 2 to 4 wt % palladium, 2 to 4 wt % silver, 5 to 9 wt % rhenium, and 0.2 to 0.6 wt % iron.

3. A process for the production of 1,4-butanediol comprising catalytically hydrogenating a hydrogenatable precursor selected from the group consisting of maleic acid, maleic anhydride, fumaric acid, succinic acid, succinic anhydride, maleate esters, succinate esters, gamma-butyrolactone and mixtures thereof, in contact with a hydrogen-containing gas and a hydrogenation catalyst comprising palladium, silver, rhenium and iron on a carbon support wherein the catalyst comprises between 0.1 to 20 wt % palladium, between 0.1 to 20 wt % silver, between 0.1 to 20 wt % rhenium, and between 0.1 to 5 wt % iron and wherein said process is conducted at a temperature of 50° C to 350° C, a hydrogen-containing gas pressure of between 20 and 400 atmospheres, a ratio of hydrogen to hydrogenatable precursor of between 5 to 1 and 1000 to 1, and a contact time of between 0.1 minute and 20 hours.

4. A process as claimed in claim 3 wherein the hydrogenatable precursor is at least one of maleic acid, succinic acid, or gamma-butyrolactone.

5. A process as claimed in claim 3 or claim 4 wherein the catalyst comprises 2 to 4 wt % palladium, 2 to 4 wt % silver, 5 to 9 wt % rhenium, and 0.2 to 0.6 wt % iron.

6. A method for making the catalyst as claimed in claims 1 or 2 or the catalyst for use in the process of any one of claims 3 to 5 comprising:
(i) oxidizing a carbon support by contacting the carbon support with an oxidizing agent;
(ii) impregnating in one or more impregnation steps comprising contacting a carbon support with a source of palladium, silver, rhenium and iron;
(iii) drying the impregnated carbon support to remove solvent after each impregnation step; and
(iv) heating the impregnated carbon support from ambient temperature to a temperature of between 100°C and 350°C under reducing conditions.

7. A method as claimed in claim 6 wherein the carbon support is contacted with an oxidizing agent at approximately the same time as the impregnation of the carbon support with the source of the palladium, silver, rhenium and iron.

8. A method as claimed in claim 6 or claim 7 wherein the oxidizing agent is selected from the group consisting of nitric acid, hydrogen peroxide, sodium hypochlorite, ammonium persulfate, perchloric acid, and oxygen.

9. A method as claimed in any one of claims 6 to 8 wherein after step (iv) the catalyst is contacted with a hydrogenatable precursor selected from the group consisting of maleic acid, maleic anhydride, fumaric acid, succinic acid, succinic anhydride, dimethyl succinate, gamma-butyrolactone and mixtures thereof, and hydrogen and heated from ambient temperature to between 40°C and 250°C.

10. A process for the production of tetrahydrofuran and 1,4-butanediol comprising catalytically hydrogenating a hydrogenatable precursor selected from the group consisting of maleic acid, maleic anhydride, fumaric acid, succinic acid, succinic anhydride, dimethyl succinate, gamma-butyrolactone and mixtures thereof in contact with a catalyst according to claim 1 or claim 2 as prepared by the method of any one of claims 6 to 9 and wherein said process is conducted at a temperature of 50° C to 350° C, a hydrogen-containing gas pressure of between 20 and 400 atmospheres, a ratio of hydrogen to hydrogenatable precursor of between 5 to 1 and 1000 to 1, and a contact time of between 0.1 minute and 20 hours.

11. A process as claimed in claim 10 wherein the hydrogenatable precursor is at least one of maleic acid, succinic acid, or gamma-butyrolactone.

12. A process as claimed in claim 10 or claim 11 wherein the catalyst comprises 2 to 4 wt % palladium, 2 to 4 wt % silver, 5 to 9 wt % rhenium, and 0.2 to 0.6 wt % iron.

13. A process as claimed in any one of claims 10 to 12 wherein the oxidizing agent is selected from the group consisting of nitric acid, hydrogen peroxide, sodium hypochlorite, ammonium persulfate, perchloric acid and oxygen.

## Revendications

1. Catalyseur consistant essentiellement en palladium, argent, rhénium et fer sur un support en carbone, ledit catalyseur comprenant 0,1 à 20% en poids de palladium, 0,1 à 20% en poids d'argent, 0,1 à 20% en poids de rhénium et 0,1 à 5% en poids de fer.

2. Catalyseur suivant la revendication 1, ledit catalyseur comprenant 2 à 4% en poids de palladium, 2 à 4% en poids d'argent, 5 à 9% en poids de rhénium et 0,2 à 0,6% en poids de fer.

3. Procédé pour la production de 1,4-butanediol, comprenant l'hydrogénation catalytique d'un précurseur hydrogénable choisi dans le groupe consistant en l'acide maléique, l'anhydride maléique, l'acide fumarique, l'acide succinique, l'anhydride succinique, des esters consistant en maléates, des esters consistant en succinates, la gamma-butyrolactone et leurs mélanges, en contact avec un gaz contenant de l'hydrogène et un catalyseur d'hydrogénation comprenant du palladium, de l'argent, du rhénium et du fer sur un support en carbone, dans lequel le catalyseur comprend 0,1 à 20% en poids de palladium, 0,1 à 20% en poids d'argent, 0,1 à 20% en poids de rhénium et 0,1 à 5% en poids de fer, ledit procédé étant mis en oeuvre à une température comprise dans l'intervalle de 50°C à 350°C, une pression du gaz contenant de l'hydrogène comprise dans l'intervalle de 20 à 400 atmosphères, un rapport, de l'hydrogène au précurseur hydrogénable compris dans l'intervalle de 5:1 à 1000:1, et un temps de contact compris dans l'intervalle de 0,1 minute à 20 heures.

4. Procédé suivant la revendication 3, dans lequel le précurseur hydrogénable est au moins un des composés consistant en acide maléique, acide succinique et gamma-butyrolactone.

5. Procédé suivant la revendication 3 ou la revendication 4, dans lequel le catalyseur comprend 2 à 4% en poids de palladium, 2 à 4% en poids d'argent, 5 à 9% en poids de rhénium et 0,2 à 0,6 % en poids de fer.

6. Procédé pour la préparation du catalyseur suivant la revendication 1 ou 2 ou du catalyseur destiné à être utilisé dans le procédé suivant l'une quelconque des revendications 3 à 5, comprenant :
(i) l'oxydation d'un support en carbone en mettant en contact le support en carbone avec un agent oxydant ;
(ii) une imprégnation dans une ou plusieurs étapes d'imprégnation, comprenant la mise en contact d'un support en carbone avec une source de palladium, d'argent, de rhénium et de fer ;
(iii) le séchage du support en carbone imprégné pour éliminer le solvant après chaque étape d'imprégnation ; et
(iv) le chauffage du support en carbone imprégné de la température ambiante à une température comprise dans l'intervalle de 100°C à 350°C dans des conditions réductrices.

7. Procédé suivant la revendication 6, dans lequel le support en carbone est mis en contact avec un agent oxydant approximativement en même temps que l'imprégnation du support en carbone avec la source de palladium, d'argent, de rhénium et de fer.

8. Procédé suivant la revendication 6 ou la revendication 7, dans lequel l'agent oxydant est choisi dans le groupe consistant en acide nitrique, peroxyde d'hydrogène, hypochlorite de sodium, persulfate d'ammonium, acide perchlorique et oxygène.

9. Procédé suivant l'une quelconque des revendications 6 à 8, dans lequel, après l'étape 4, le catalyseur est mis en contact avec un précurseur hydrogénable choisi dans le groupe consistant en acide maléique, anhydride maléique, acide fumarique, acide succinique, anhydride succinique, succinate de diméthyle, gamma-butyrolactone et leurs mélanges, et de l'hydrogène, et est chauffé de la température ambiante à une température comprise dans l'intervalle de 40°C à 250°C.

10. Procédé pour la production de tétrahydrofuranne et de 1,4-butanediol, comprenant l'hydrogénation catalytique d'un précurseur hydrogénable choisi dans le groupe consistant en acide maléique, anhydride maléique, acide fumarique, acide succinique, anhydride succinique, succinate de diméthyle, gamma-butyrolactone et leurs mélanges en contact avec un catalyseur suivant la revendication 1 ou 2 préparé par le procédé suivant l'une quelconque des revendications 6 à 9, ledit procédé étant mis en oeuvre à une température comprise dans l'intervalle de 50°C à 350°C, une pression du gaz contenant de l'hydrogène comprise dans l'intervalle de 20 à 400 atmosphères, un rapport de l'hydrogène au précurseur hydrogénable compris dans l'intervalle de 5:1 à 1000:1 et un temps de contact compris dans l'intervalle de 0,1 minute à 20 heures.

11. Procédé suivant la revendication 10, dans lequel le précurseur hydrogénable est au moins un des composés consistant en acide maléique, acide succinique et gamma-butyrolactone.

12. Procédé suivant la revendication 10 ou , la revendication 11, dans lequel le catalyseur comprend 2 à 4% en poids de palladium, 2 à 4% en poids d'argent, 5 à 9% en poids de rhénium et 0,2 à 0,6% en poids de fer.

13. Procédé suivant l'une quelconque des revendications 10 à 12, dans lequel l'agent oxydant est choisi dans le groupe consistant en acide nitrique, peroxyde d'hydrogène, hypochlorite de sodium, persulfate d'ammonium, acide perchlorique et oxygène.

## Patentansprüche

1. Katalysator, der im Wesentlichen aus Palladium, Silber, Rhenium und Eisen auf einem Kohlenstoff-Träger besteht, wobei der Katalysator 0,1 bis 20 Gew.-% Palladium, 0,1 bis 20 Gew.-% Silber, 0,1 bis 20 Gew.-% Rhenium und 0,1 bis 5 Gew.-% Eisen umfasst.

2. Katalysator nach Anspruch 1, wobei der Katalysator 2 bis 4 Gew.-% Palladium, 2 bis 4 Gew.-% Silber, 5 bis 9 Gew.-% Rhenium und 0,2 bis 0,6 Gew.-% Eisen umfasst.

3. Verfahren zur Produktion von 1,4-Butandiol, welches umfasst, dass man katalytisch eine hydrierbare Vorstufe, die aus der Gruppe ausgewählt ist, die aus Maleinsäure, Maleinsäureanhydrid, Fumarsäure, Bernsteinsäure, Bernsteinsäureanhydrid, Maleatestern, Succinatestern, gamma-Butyrolacton und deren Mischungen besteht, in Kontakt mit einem Wasserstoff-haltigen Gas und einem Hydrierkatalysator hydriert, welcher Palladium, Silber, Rhenium und Eisen auf einem Kohlenstoff-Träger umfasst, wobei der Katalysator 0,1 bis 20 Gew.-% Palladium, 0,1 bis 20 Gew.-% Silber, 0,1 bis 20 Gew.-% Rhenium und 0,1 bis 5 Gew.-% Eisen umfasst und wobei das Verfahren bei einer Temperatur von 50°C bis 350°C, einem Druck des Wasserstoff-haltigen Gases von 20 bis 400 Atmosphären, einem Verhältnis von Wasserstoff zu hydrierbarer Vorstufe von 5 zu 1 bis 1000 zu 1 und einer Kontaktzeit von 0,1 Minuten bis 20 Stunden durchgeführt wird.

4. Verfahren nach Anspruch 3, in dem die hydrierbare Vorstufe mindestens eine aus Maleinsäure, Bernsteinsäure oder gamma-Butyrolacton ist.

5. Verfahren nach Anspruch 3 oder Anspruch 4, in dem der Katalysator 2 bis 4 Gew.-% Palladium, 2 bis 4 Gew.-% Silber, 5 bis 9 Gew.-% Rhenium und 0,2 bis 0,6 Gew.-% Eisen umfasst.

6. Verfahren zur Herstellung des Katalysators nach Anspruch 1 oder 2 oder des Katalysators zur Verwendung in dem Verfahren nach irgendeinem der Ansprüche 3 bis 5, welches umfasst:
(i) Oxidieren eines Kohlenstoff-Trägers durch Kontaktieren des Kohlenstoff-Trägers mit einem Oxidationsmittel;
(ii) Imprägnieren in einem oder mehreren Imprägnierungsschritten, welches umfasst, dass man einen Kohlenstoff-Träger mit einer Quelle von Palladium, Silber, Rhenium und Eisen in Kontakt bringt;
(iii) Trocknen des imprägnierten Kohlenstoff-Trägers nach jedem Imprägnierungsschritt, um Lösungsmittel zu entfernen; und
(iv) Erwärmen des imprägnierten Kohlenstoff-Trägers von Umgebungstemperatur auf eine Temperatur zwischen 100°C und 350°C unter reduzierenden Bedingungen.

7. Verfahren nach Anspruch 6, in dem der Kohlenstoff-Träger zu etwa derselben Zeit wie der Imprägnierung des Kohlenstoff-Trägers mit der Quelle des Palladiums, Silbers, Rheniums und Eisens mit einem Oxidationsmittel in Kontakt gebracht wird.

8. Verfahren nach Anspruch 6 oder Anspruch 7, in dem das Oxidationsmittel aus der Gruppe ausgewählt ist, die aus Salpetersäure, Wasserstoffperoxid, Natriumhypochlorit, Ammoniumpersulfat, Perchlorsäure und Sauerstoff besteht.

9. Verfahren nach irgendeinem der Ansprüche 6 bis 8, in dem nach dem Schritt (iv) der Katalysator mit einer hydrierbaren Vorstufe, die aus der Gruppe ausgewählt ist, die aus Maleinsäure, Maleinsäureanhydrid, Fumarsäure, Bernsteinsäure, Bernsteinsäureanhydrid, Dimethylsuccinat, gamma-Butyrolacton und deren Mischungen besteht, und Wasserstoff in Kontakt gebracht wird und von Umgebungstemperatur auf zwischen 40°C und 250°C erwärmt wird.

10. Verfahren zur Produktion von Tetrahydrofuran und 1,4-Butandiol, welches umfasst, dass man eine hydrierbare Vorstufe, die aus der Gruppe ausgewählt ist, die aus Maleinsäure, Maleinsäureanhydrid, Fumarsäure, Bernsteinsäure, Bernsteinsäureanhydrid, Dimethylsuccinat, gamma-Butyrolacton und deren Mischungen besteht, in Kontakt mit einem Katalysator nach Anspruch 1 oder Anspruch 2, wie durch das Verfahren nach irgendeinem der Ansprüche 6 bis 9 hergestellt, katalytisch hydriert, und wobei das Verfahren bei einer Temperatur von 50°C bis 350°C, einem Druck von Wasserstoff-haltigem Gas zwischen 20 und 400 Atmosphären, einem Verhältnis von Wasserstoff zu hydrierbarer Vorstufe zwischen 5 zu 1 und 1000 zu 1 und einer Kontaktzeit zwischen 0,1 Minuten und 20 Stunden durchgeführt wird.

11. Verfahren nach Anspruch 10, in dem die hydrierbare Vorstufe mindestens eine aus Maleinsäure, Bernsteinsäure oder gamma-Butyrolacton ist.

12. Verfahren nach Anspruch 10 oder Anspruch 11, in dem der Katalysator 2 bis 4 Gew.-% Palladium, 2 bis 4 Gew.-% Silber, 5 bis 9 Gew.-% Rhenium und 0,2 bis 0,6 Gew.-% Eisen umfasst.

13. Verfahren nach irgendeinem der Ansprüche 10 bis 12, in dem das Oxidationsmittel aus der Gruppe ausgewählt ist, die aus Salpetersäure, Wasserstoffperoxid, Natriumhypochlorit, Ammoniumpersulfat, Perchlorsäure und Sauerstoff besteht.
